(19) Europäisches Patentamt
European Patent Office
Office européen des brevets

(11) Publication number: **0 069 513**
**B1**

(12) **EUROPEAN PATENT SPECIFICATION**

(45) Date of publication of patent specification: **04.03.87**

(51) Int. Cl.[4]: **C 07 D 401/06,**
C 07 D 403/06, A 61 K 31/44,
A 61 K 31/415

(21) Application number: **82303313.9**

(22) Date of filing: **25.06.82**

(54) **Indole thromboxane synthetase inhibitors, processes for their preparation, and pharmaceutical compositions containing them.**

(30) Priority: **07.07.81 GB 8120878**
**13.05.82 GB 8213864**

(43) Date of publication of application:
**12.01.83 Bulletin 83/02**

(45) Publication of the grant of the patent:
**04.03.87 Bulletin 87/10**

(84) Designated Contracting States:
**BE DE FR GB IT LU NL**

(56) References cited:
**EP-A-0 003 901**
**EP-A-0 054 417**
**DE-A-1 901 167**
**FR-A-2 272 081**
**GB-A-2 045 244**
**US-A-3 445 472**

**Chemical Abstracts vol. 95, no. 15, 12 October 1981, Columbus, Ohio (US); T.TANOUCHI et al.: "Highly selective inhibitors ofthromboxane synthetase. 2 . Pyridine derivatives", p. 20, abstract 125868g**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE DE FR IT LU NL**

(72) Inventor: **Cross, Peter Edward**
**21 Cherry Avenue**
**Canterbury Kent (GB)**
Inventor: **Dickinson, Roger Peter**
**6 Kingston Close**
**River Dover Kent (GB)**

(74) Representative: **Wood, David John et al**
**Pfizer Limited Ramsgate Road**
**Sandwich Kent CT13 9NJ (GB)**

**EP 0 069 513 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to indole derivatives, and in particular to certain 3-(pyridylmethyl)- indolyl acrylic acid derivatives. Such compounds are able to selectively inhibit the action of the thromboxane synthetase enzyme without significantly inhibiting the action of the prostacyclin synthetase or cyclo-oxygenase enzymes. The compounds may thus be useful in, for example, the treatment of thombosis, ischaemic heart disease, stroke, transient ischaemic attack, migraine, cancer and the vascular complications of diabetes.

Thus, according to the invention there are provided compounds of the general formula:

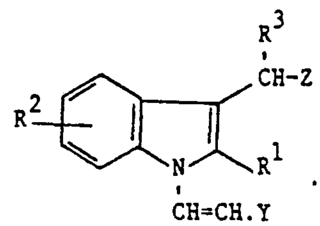

(I)

wherein

$R^1$ is hydrogen or $C_1$—$C_4$ alkyl;

$R^2$ is hydrogen, $C_1$—$C_4$ alkyl, $C_1$—$C_4$ alkoxy or halo;

$R^3$ is hydrogen or methyl:

Y is —COOH, —COO($C_1$—$C_4$ alkyl) or —$CONH_2$; and

Z is 3-pyridyl;

and the pharmaceutically acceptable salts thereof.

"Halo" means F, Cl, Br or I.

Alkyl and alkoxy groups of 3 and 4 carbon atoms may be straight or branched chain.

The preferred compounds are in the E (trans) form, i.e. where the group "Y" is trans to the indole ring. $R^3$ is preferably H. Preferably $R^1$ is $CH_3$ and Y is —COOH. $R^2$ is preferably H, $CH_3$, $OCH_3$ or Cl. When $R^2$ is a substituent, it is preferably in the 5-position.

In the most preferred compounds, $R^1$ is $CH_3$, $R^2$ and $R^3$ are hydrogen and Y is —COOH.

The invention further provides a compound of the formula (I), or a pharmaceutically acceptable salt thereof, for use in treating an animal, including a human being, to inhibit the action of the thromboxane synthetase enzyme without significantly inhibiting the action of the prostacyclin synthetase or cyclo-oxygenase enzymes.

The invention also includes a pharmaceutical composition comprising a compound of the formula (I), or a pharmaceutically acceptable salt thereof, together with a pharmaceutically acceptable diluent or carrier.

The preferred salts are the pharmaceutically acceptable acid addition, and, when Y is —COOH, the pharmaceutically acceptable metal or ammonium salts.

Pharmaceutically acceptable acid addition salts of the compounds of the invention are salts with acids containing pharmaceutically acceptable anions, e.g. the hydrochloride, hydrobomide, sulphate or bisulphate, phosphate or acid phosphate, acetate, maleate, fumarate, lactate, tartrate, citrate, gluconate, succinate and p-toluene sulphonate salts.

The preferred metal salts are the alkali metal salts.

(1) The compounds of the formula (I) may be prepared by a number of different routes, including by the following reaction scheme:—

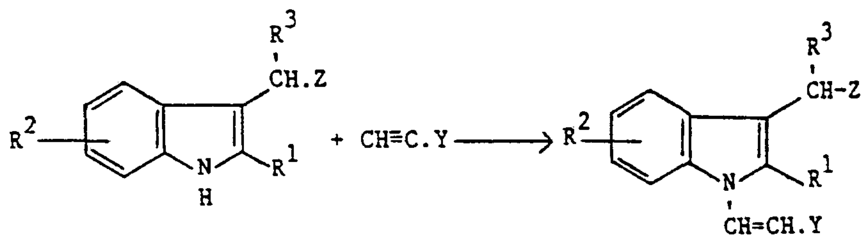

(II) (III) (I)

where $R^1$, $R^2$, $R^3$, Y and Z are as defined for formula (I).

In a typical procedure, the indole (II) and alkyne (III) are reacted together preferably in the presence of a base (e.g. benzyltrimethylammonium hydroxide or tetrabutylammonium fluoride) and in a suitable organic solvent, e.g. dioxan or tetrahydrofuran, typically for a few hours, e.g. up to 4 hours, at room temperature, although the reaction mixture may be heated, e.g. at up to 100°C, to accelerate the reaction.

The product can be isolated and purified by conventional procedures as are illustrated in the following Examples.

This route prepares products in the E-form: products in the Z (cis) form are generally obtainable by irradiation of the E-form with u.v. light according to conventional procedures.

The starting materials of the formulae (II) and (III) are either known compounds or can be prepared by procedures analogous to those of the prior art. The starting materials can be prepared by the following routes:—

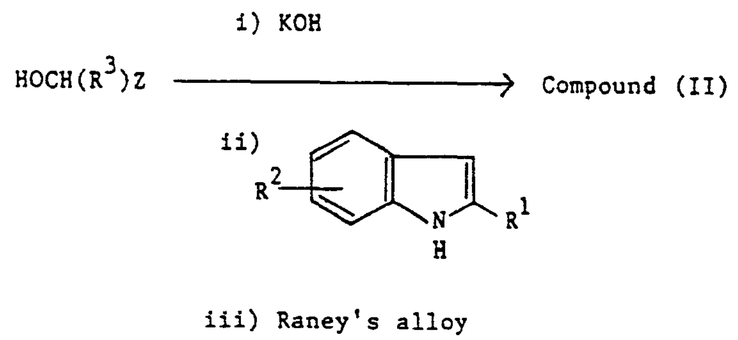


or

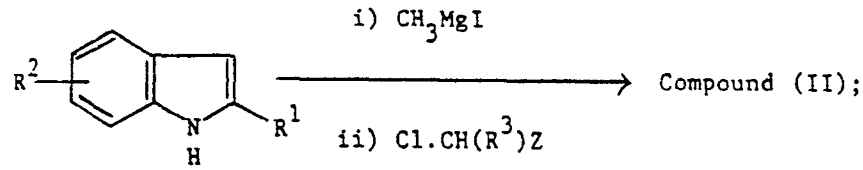


the starting indoles being described in European patent no. 3901.

Pyridyl intermediates in which $R^3$ is $C_1$—$C_4$ alkyl can also be prepared by the following route:—

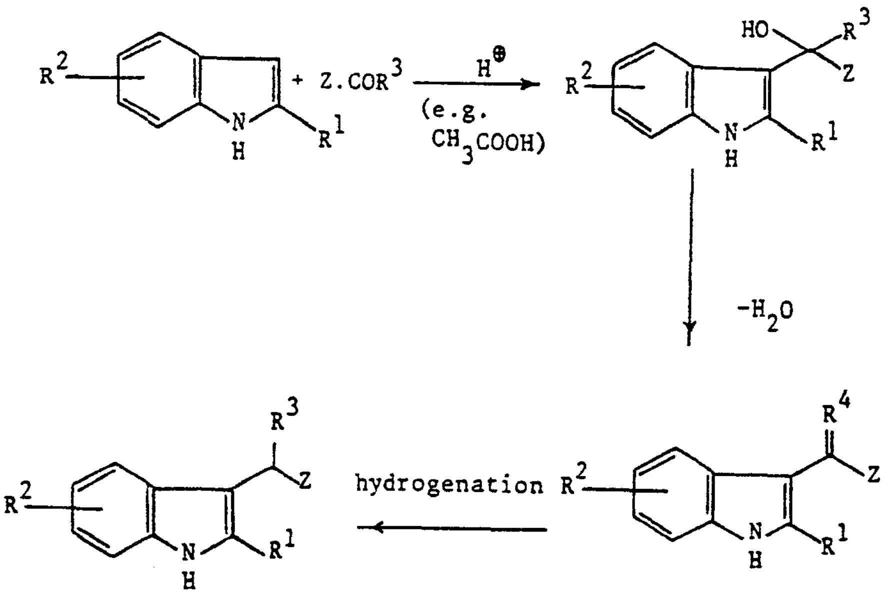


$R^1$, $R^2$ and $R^3$ are as defined for formula (I).

$R^4$ is the alkylene group having the same number of carbon atoms as $R^3$. For example, the dehydration of the intermediate in which $R^3$ is $CH_3$ yields the compound in which $R^4$ is $CH_2$, which compound is then hydrogenated (using e.g. Pd/C, $C_2H_5OH$, $H_2$ at 2—5 atm.) to the desired end product.

(2) Naturally certain of the groups Y can be obtained by chemical transformation reactions and these possibilities will be well known to those skilled in the art. Thus, for example, compounds for the formula (I) wherein Y is a carboxyl group can be obtained by the hydrolysis, preferably alkaline hydrolysis, of the corresponding esters where Y is $—COO(C_1—C_4$ alkyl). The acid may be converted to a variety of derivatives, e.g. formation of the acid chloride or bromide or the imidazolide followed by reaction with ammonia gives the amides where Y is $CONH_2$. Also, the esters in which Y is $—COO(C_1—C_4$ alkyl) can be reacted with ammonia to form the corresponding amides.

All these reactions are entirely conventional and the methods and conditions for their performance will be well known to those skilled in the art, as with other possibilities and variations.

The pharmaceutically acceptable acid addition salts of the compounds of the invention can be prepared by conventional procedures, e.g. by reacting the free base in a suitable solvent, e.g. ethanol, with a solution containing one equivalent of the desired acid in a suitable solvent, e.g. ether. The salt generally precipitates from solution or is recovered by evaporation of the solvent. Similarly the pharmaceutically acceptable metal and ammoniumn salts can be prepared by conventional procedures.

Where the compounds of the invention contain an asymmetric carbon atom the invention includes the racemic mixtures and the separated D- and L- optically active isomeric forms. Such forms should be obtainable by conventional methods, e.g. by fractional crystallisation of a salt with a suitable optically active acid, e.g. tartaric acid.

The compounds of formula (I) and their pharmaceutically acceptable salts have been found to selectively inhibit the action of the thromboxane synthetase enzyme without significantly affecting the action of the prostacyclin synthetase or cyclooxygenase enzymes. Thus the compounds are of value in the teatment of a variety of clinical conditions which are characterised by an imbalance of prostacyclin/thromboxane $A_2$. For the reasons given below these conditions may include thombosis, ischaemic heart disease, stroke, transient ischaemic attack, migraine, cancer and the vascular complications of diabetes.

Research work has established that in most tissues the major product of the arachidonic acid metabolism is either of two unstable substances, thromboxane $A_2$ ($TxA_2$) or prostacyclin ($PGI_2$). (Proc. Nat. Acad. Sci. U.S.A., 1975, *72,* 2994, Nature, 1976, *263,* 663, Prostaglandins, 1976, *12,* 897). In most cases the prostaglandins $PGE_2$, $PGF_2$ and $PGD_2$ are comparatively minor by-products in this bio-synthetic pathway. The discovery of thromboxane $A_2$ and prostacyclin has significantly increased our understanding of vascular homeostasis, prostacyclin for instance is a powerful vasodilator and inhibitor of platelet aggregation, and in this last respect is the most potent endogenous substance so far discovered. The prostacyclin synthetase enzyme is located in the endothelial layer of the vasculature, and may be fed by endoperoxides released by blood platelets coming into contact with the vessel wall. The prostacyclin thus produced is important for prevention of platelet deposition on vessel walls. (Prostaglandins, 1976, *12,* 685, Science, 1976, 17, Nature, 1978, *273,* 765).

Thromboxane $A_2$ is synthesised by the thomboxane synthetase enzyme which is located in, for example, the blood platelets. Thromboxane $A_2$ is a powerful vasoconstrictor and pro-aggregatory substance. As such its actions are in direct opposition to those of prostacyclin. If, for any reason, prostacyclin formation by the vasculature is impaired, then the endoperoxides produced by platelets coming into contact with the vessel wall may be converted into thromboxane, but are not converted effectively into prostacyclin (Lancet, 1977, 18, Prostaglandins, 1978, *13,* 3). Alteration of the prostacyclin/thomboxane balance in favour of the latter substance could result in platelet aggregation, vasospasm (Lancet, 1977, 479, Science, 1976, Amer. J. Cardiology, 1978, *41,* 787) and an increased susceptibility to atherothrombosis (Lancet (i) 1977, 1216). It is also known that in experimental atherosclerosis prostacyclin generation is suppressed and thomboxane $A_2$ production is enhanced (Prostaglandins, 1977, *14,* 1025 and 1035). Thus thromboxane $A_2$ has been implicated as the causative agent in variant angina, myocardial infarction, sudden cardiac death and stroke (Thromb. Haemostasis, 1977, *38,* 132). Studies in rabbits have shown that ECG changes typical of these conditions were produced when freshly prepared thromboxane $A_2$ was injected directly into the animal's heart (Biochem. aspects of Prostaglandins and Thromboxanes, Editors, N. Kharasch and J. Fried. Academic Press 1977 page 189). This technique is considered to represent a unique animal model of the heart attacks of coronary patients and has been used to show that administration of a compound believed to antagonise the effects of thromboxane $A_2$ protects the rabbits from the adverse consequences of thromboxane $A_2$ injection.

Another area where a $PGI_2/TxA_2$ imbalance is considered to be a contributory factor is that of migraine. The migraine headache is associated with changes in intra and extracerebral blood flow, in particular a pre-headache reduction of cerebral blood flow followed by dilation in both vascular areas during the headache phase.

Prior to the development of the headache, blood levels of 5-hydroxytryptamine are elevated, and this suggests the occurrence of *in vivo* aggregation and release of the amine from the platelet stores. It is known that the blood platelets of migraine patients are more prone to aggregate than are those of normal individuals (J. Clin. Pathol., 1971, *24,* 250, J. Headache, 1977, *17,* 101). Furthermore, it has now been postulated that not only is an abnormality of platelet function a major factor in the pathogenesis of migraine attacks but it is in fact their prime cause (Lancet (i), 1978, 501). Thus a drug that selectively modifies platelet function to inhibit thromboxane $A_2$ formation could be of considerable benefit in migraine therapy.

Abnormalities of platelet behaviour have been reported in patients with diabetes mellitus (Metabolism, 1979, *28,* 394, Lancet, 1978 (i) 235). Diabetic patients are known to be particularly susceptible to microvascular complications, atherosclerosis and thrombosis and platelet hyper-reactivity has been suggested as the cause of such angiopathy. Diabetic platelets produce elevated amounts of $TxB_2$ and malondialdehyde (Symposium "Diabetes and Thrombosis — Implications for Therapy", Leeds U.K., April 1979). Also it has been shown that in rats with experimental diabetes vascular prostacyclin production is impaired and $TxA_2$ synthesis from the platelets is elevated (IV International Prostaglandin Conference, Washington, D.C., May 1979). Thus the imbalance between prostacyclin and $TxA_2$ is considered to be responsible for the microvascular complications of diabetes. A $TxA_2$-synthetase inhibitor could therefore find clinical utility in preventing these vascular complications.

Aspirin and most other non-steroidal anti-inflammatory drugs inhibit the cyclo-oxygenase enzyme. The effect of this is to shut down the production of the $PGG_2/H_2$ endoperoxides and by so doing to reduce both the prostacyclin and thromboxane $A_2$ levels. Aspirin and aspirin-like drugs have been evaluated clinically for prevention of stroke and heart attack (New England and J. Med. 1978, *299,* 53, B.M.J., 1978, 1188, Stroke, 1977, *8,* 301).

Although some encouraging results have been obtained with these drugs, a compound which specifically inhibits thromboxane $A_2$ formation leaving the biosynthesis of prostacyclin unimpaired would be more valuable in these clinical conditions (Lancet (ii), 1978, 780).

The ability of primary neoplasms to metastasize is a principal cause of failure to cure human cancers. It has been suggested that metastatic tumour cells can alter the critical $PGI_2$-$TxA_2$ balance in favour of thrombosis (Science, 1981, *212,* 1270). Prostacyclin has recently been shown to be a powerful antimetastatic agent by virtue of its platelet antiaggregatory action. This result indicates that a $TxA_2$-synthetase inhibitor would function as an antimetastatic agent *in vivo* (J. Cell. Biol. 1980, *87* 64).

The effect of the compounds of the formula (I) on the thromboxane synthetase enzyme, and the prostacyclin synthetase and cyclooxygenase enzymes has been measured by the following *in vivo* enzyme assays:—

1. Cyclo-oxygenase

Ram seminal vesicle microsomes (Biochemistry, 1971, *10,* 2372) are incubated with arachidonic acid (100 µM: 1 min.: 22°) to produce $PGH_2$ and aliquots of the reaction mixture injected into a stream of Krebs-bicarbonate at 37°C containing a mixture of antagonists (Nature, 1978, *218,* 1135) and indomethacin (Brit. J. Pharmacol., 1972, *45* 451) which is superfusing a spirally-cut rabbit aorta strip (Nature, 1969, *223,* 29).

The ability of a compound to inhibit the enzyme is measured by comparing the increases in isometric tension produced by $PGH_2$ in the absence of the test compound, and following pre-incubation of the enzyme with the test compound for 5 minutes (Agents and Actions, 1981, *11,* 274).

2. Prostacyclin ($PGI_2$) Synthetase

Pig aorta microsomes (Nature, 1976, *263,* 663) are incubated (30 sec.: 22°C) with $PGH_2$ produced as in 1 and aliquots bio-assayed as in 1. $PGI_2$ production is assessed indirectly by measuring the decrease in $PGH_2$-induced tension ($PGI_2$ itself does not contract the aorta). This decrease can be prevented completely by pre-incubation of the enzyme with the selective $PGI_2$ synthetase inhibitor, 15-hydroxy-arachidonic acid (Prostaglandins, 1976, *12,* 715). The test compounds is then pre-incubated with the enzyme for 5 minutes, and its ability to prevent the decrease in tension is measured.

3. Thromboxane $A_2$ ($TxA_2$) Synthetase

Indomethacin pre-treated human platelet microsomes (Science, 1976, *193,* 163) are incubated (2 min.: 0°C) with $PGH_2$ (produced as in 1) and aliquots of the reaction mixture superfused over two rabbit aorta spirals which are separated by a delay coil (2 min.). The latter is required to allow the selective decay of the more unstable thromboxane $A_2$ (Proc. Nat. Acad. Sci., 1975, *72,* 2994) thereby enabling the separate measurement of increased isometric tension due to the $TxA_2$ formed and the $PGH_2$ remaining. The test compound is pre-incubated with enzyme for 5 minutes, and its ability to inhibit the thromboxane synthetase enzyme is measured as its reduction of the $TxA_2$ component of the isometric tension.

Compounds of the formula (I) tested in this way have been shown to be capable of selectively inhibiting the thromboxane synthetase enzyme.

In addition to the above an *in vitro* assay for measuring the inhibition of human blood platelet aggregation has been described and this may be predictive of anti-thrombotic efficacy clinically (Lancet (ii), 1974, 1223, J. Exp. Med., 1967, *126,* 171). Both clinically effective agents aspirin and sulphinpyrazone show inhibitory activity *in vitro* against a variety of aggregating agents in this test.

A number of *in vivo* tests in animals have also been described for evaluating potential anti-thrombotic drugs.

The method of Patrono *et al* is adapted to study the generation of $TxB_2$ in whole blood samples removed from animals prior to and following drug treatment. Briefly, blood samples are taken into glass tubes and allowed to clot at 37°C. Serum is separated by centrifugation and the samples stored at −40°C until assayed for $TxB_2$, when appropriate dilutions of ethanol deproteinised samples are analysed by RIA. This technique is used in experiments with the test compounds to determine intravenous potency in anaesthetised rabbits:—

Anaesthetised Rabbits

Male New Zealand white rabbits (2.6—5.6 kg) are anaesthetised with sodium pentobarbitone (30 mg/kg i.v.) followed by urethane (500 mg/kg i.p). After cannulation of the trachea, a carotid artery is catheterised for collection of blood samples. The catheter is kept patent by slow infusion (0.2 ml/minute) of sterile saline. Control carotid arterial blood samples are taken 30 and 5 minutes prior to administration of the test compound or vehicle (0.9% w/v NaCl, 0.2 ml/kg) via a marginal ear vein. Three groups of rabbits are used. The first group receive 0.03 mg/kg of the test compound followed, one hour later, by 0.1 mg/kg. Similarly, the second group receive 0.3 mg/kg, followed by 1 mg/kg. The third group receive vehicle, followed one hour later by a further vehicle injection. Carotid arterial blood samples are taken at various times after all doses. At each time point, a 1 ml blood sample is taken into a glass test tube, without anticoagulant, for $TxB_2$ determination. For the latter, the blood sample is allowed to clot during a two hour incubation at 37°C (which preliminary experiments had shown to give maximum $TxB_2$ production) and the serum obtained by centrifugation. Serum samples are then processed through the $TxB_2$ RIA after deproteinisation with ethanol and dilution with Isogel Tris buffer.

Intravenous injection of arachidonic acid causes death in rabbits by causing platelet clumping and embolisation in the lungs. Again both the clinically effective aspirin (Agents and Actions, 1977, 1, 481) and sulphinpyrazone (Pharmacology, 1976, *14,* 522) protect the rabbit from the lethal effect of the injection. Sulphinpyrazone has also been shown to prevent the aggregation of platelets in an extra corporeal loop of the abdominal aorta of rats *in vivo* (Thromb. Diathes. Haem., 1973, *30,* 138).

The compounds may be administered orally in the form of tablets of capsules containing a unit dose of the compound together with such excipients as maize starch, calcium carbonate, dicalcium phosphate, alginic acid, lactose, magnesium stearate, "Primogel" (Trade Mark) or talc. The tablets are typically prepared by granulating the ingredients together and compressing the resulting mixture to give tablets of the desired size. Capsules are typically prepared by granulating the ingredients together and filling them into hard gelatine capsules of the appropriate size to obtain the desired dosage.

The compounds may also be administered parenterally, for example by intramuscular, intravenous or subcutaneous injection. For parenteral administration, they are best used in the form of a sterile aqueous solution which may contain other solutes such as tonic and pH adjusters. The compounds may be added to distilled water and the pH adjusted to 3—6 using an acid such as citric, lactic or hydrochloric acid. Sufficient solutes such as dextrose or saline may be added to render the solution isotonic. The resulting solution may then be sterilised and filled into sterile glass vials of an appropriate size to contain the desired volume of solution. The compounds of the invention may also be administered by the infusion of a parenteral formulation as described above into a vein.

For oral administration to human patients, it is expected that the daily dosage level of a compound of the formula (I) will be from 0.1 to 20 mg/kg per day for a typical adult patient (70 kg). For parenteral administration, it is expected that the daily dosage level of a compound of the formula (I) will be from 0.01—0.5 mg/kg. per day, for a typical adult patient. Thus tablets or capsules can generally be expected to contain from 5 to 150 mg of the active compound for administration orally up to 3 times a day. Dosage units for parenteral administration can be expected to contain from 0.5—35 mg of the active compound A typical vial could be a 10 ml vial containing 5 mg of the active compound in 6—10 ml of solution.

It should of course be appreciated that in any event the physician will determine the actual dosage which will be most suitable for the individual and it will vary with the age, weight and response of the patient.

The above dosages are exemplary of the average patient, there may of couse be individual cases where higher or lower dosage ranges are merited.

The preparation of the novel compounds of the formula (I) is illustrated by the following Examples. All temperatures are in °C.

## Example 1

E-3-{1-[2-methyl-3-(3-pyridylmethyl)]indolyl}acrylic acid

Treatment of 2-methyl-3-(3-pyridylmethyl)indole with ethyl propiolate followed by hydrolysis of the intermediate ester by a procedure similar to that described in Example 1 of EP—A—0069513, gave E-3-{1-[2-methyl-3-(3-pyridylmethyl)]indolyl}acrylic acid, m.p. 206—207° (from methanol).

Analysis %:—

| | | | | |
|---|---|---|---|---|
| Found: | C, 73.48; | H, 5.53; | N, 9.78. | $C_{18}H_{16}N_2O_2$ |
| Requires: | C, 73.95; | H, 5.52; | N, 9.58. | |

TABLE 1

The following esters were prepared by the method of Example 3 of EP—A—0069513 from appropriate starting materials

| Example No. | Compound | M.P. (°C) | Analysis % |
|---|---|---|---|
| 2 | 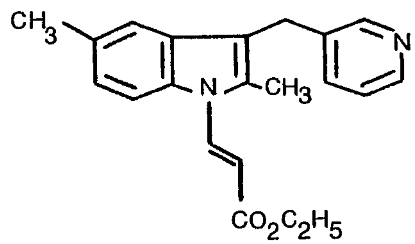 | 125—126 | Found : C, 75.20 ; H, 6.43 ; N, 8.34. $C_{21}H_{22}N_2O_2$<br>Required : C, 75.42 ; H, 6.63 ; N, 8.38. |
| 3 | 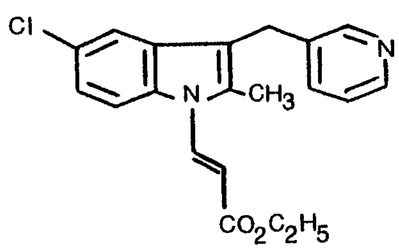 | 126—127 | Found : C, 67.55 ; H, 5.38 ; N, 8.03. $C_{20}H_{19}ClN_2O_2$<br>Required : C, 67.69 ; H, 5.40 ; N, 7.90. |
| 4 | 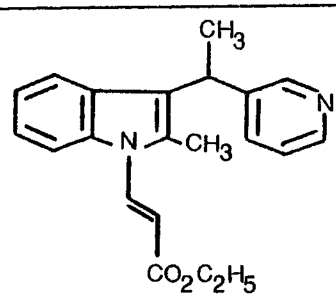 | | Product hydrolysed directly after chromatography |

Hydrolysis of the ethyl esters according to the method described in Example 1 of EP—A—0069513 gave the carboxylic acids listed in Table 2.

TABLE 2

| Example No. | Compound | M.P. (°C) | Analysis % | | |
|---|---|---|---|---|---|
| 5 | 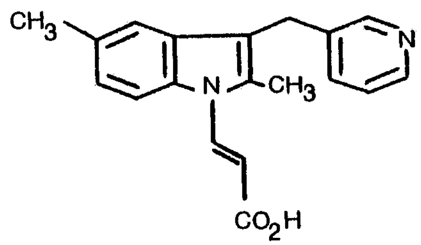 | 238—240 | Found: | C, 74.28; H, 6.00; N, 8.92. | $C_{19}H_{18}N_2O_2$ |
| | | | Required: | C, 74.49; H, 5.92; N, 9.15. | |
| 6 | 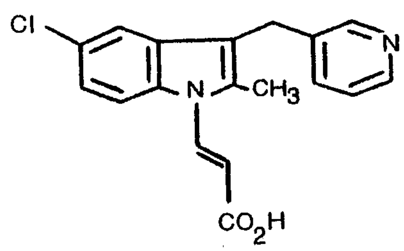 | 242—243 | Found: | C, 65.75; H, 4.34; N, 8.46. | $C_{18}H_{15}ClN_2O_2$ |
| | | | Required: | C, 66.15; H, 4.63; N, 8.57. | |
| 7 | 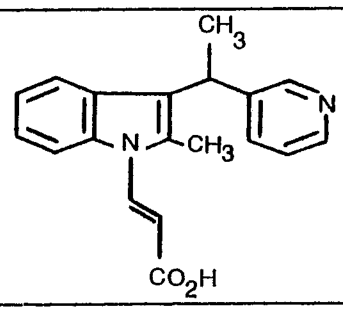 | 188—189 | Found: | C, 74.32; H, 6.21; N, 8.83. | $C_{19}H_{18}N_2O_2$ |
| | | | Required: | C, 74.49; H, 5.92; N, 9.15. | |

Example 8

E-3-{1-[5-Chloro-2-methyl-3-(3-pyridylmethyl)]indolyl}acrylamide

A mixture of E-3-{1-[5-chloro-2-methyl-3-(3-pyridylmethyl)]indolyl}acrylic acid (0.33 g), N,N'-carbonyldi-imidazole (0.20 g) and dry dioxan (5 ml) was heated on a steam bath for 2 hours and then evaporated. An excess of a concentrated solution of ammonia in ethanol was added and the solution was allowed to stand for 30 minutes and then evaporated. The gummy residue was triturated with water to give a solid which was crystallized from methanol to give E-3-{1-[5-chloro-2-methyl-3-(3-pyridylmethyl)]-indolyl}acrylamide, m.p. 262—263°C.

Analysis %:—

| | | | | |
|---|---|---|---|---|
| Found: | C, 65.88; | H, 5.23; | N, 12.44. | $C_{18}H_{16}ClN_3O$ |
| Requires: | C, 66.36; | H, 4.95; | N, 12.90. | |

The following illustrates the preparation of a starting material used in Example 1. All temperatures are in °C:—

Preparation 1

Preparation of 2-Methyl-3-(3-pyridylmethyl)indole

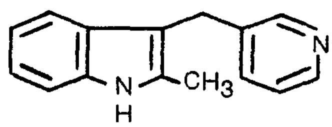

3-Pyridylmethanol (27.25 g) was added to a suspension of KOH (2.24 g) in xylene (200 ml), and the mixture heated at reflux using a Dean Stark apparatus to remove the water. After cooling, 2-methylindole (16.4 g) was added and the mixture heated at reflux for 3 hours. "Raneys Alloy" (1.0 g) was then added to the hot solution and heating at reflux was continued overnight. After cooling, the metallic residue was filtered off and washed with ether (25 ml). The combined organic filtrate was extracted with $H_2O$ (2 × 100 ml) and the organic layer separated and cooled to 0°, whereupon a solid precipitated which was filtered off. Crystallisation of the solid from toluene afforded the pure title compound, 14.6 g, m.p. 207—210°.

Analysis:—

| | | | | |
|---|---|---|---|---|
| Found: | C, 81.05; | H, 6.35; | N, 12.6. | $C_{15}H_{14}N_2$ |
| Required: | C, 80.6; | H, 6.3; | N, 12.15. | |

Preparation 2

2-Methyl-3-(1-[3-pyridyl]ethyl)indole

A solution of 1-(2-methyl-3-indolyl)-1-(3-pyridyl)ethylene (prepared according to J. Het. Chem., *9,* 833, 1972) (9.37 g) in ethanol (200 ml) was hydrogenated at 2.5 atm. pressure in the presence of 10% palladium/charcoal. The solution was filtered and evaporated and the residue was crystallised from ethyl acetate/petrol (b.p. 60—80°) to give 2-methyl-3-[1-(3-pyridyl)ethyl]indole (5.74 g) m.p. 139—141°C.

Analysis %:—

| | | | | |
|---|---|---|---|---|
| Found: | C, 81.56; | H, 7.11; | N, 11.65. | $C_{16}H_{16}N_2$. |
| Requires: | C, 81.32; | H, 6.83; | N, 11.86%. | |

ACTIVITY RESULTS

% Inhibition of $TxB_2$ formation after i.v. administration to rabbits:—

| Compound | Dose (mg/kg) | % Inhibition $T\times B_2$ Production | | | | |
|---|---|---|---|---|---|---|
| | | 2 Min | 15 Min | 30 Min | 45 Min | 75 Min |
| 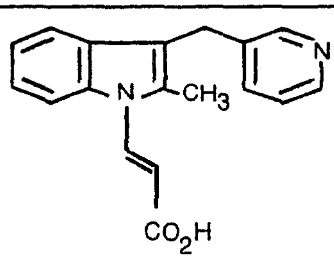 | 0.1 | | 81 | 82 | | |
| | 0.3 | 95 | 98 | | 93 | 90 |
| | 1.0 | 97 | 94 | | 97 | 95 |

**Claims**

1. A compound of the formula:—

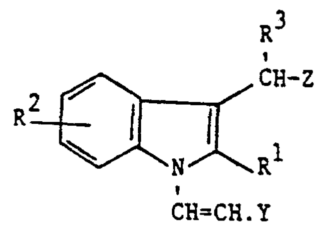

(I)

where
$R^1$ is H or $C_1$—$C_4$ alkyl;
$R^2$ is H, $C_1$—$C_4$ alkyl, $C_1$—$C_4$ alkoxy or halo;
$R^3$ is H or methyl;
Y is —COOH, —COO($C_1$—$C_4$ alkyl) or —$CONH_2$; and
Z is 3-pyridyl;
and their pharmaceutically acceptable salts.

2. A compound as claimed in claim 1, where $R^3$ is hydrogen.

3. A compound as claimed in claim 1 or 2, wherein $R^1$ is methyl and Y is —COOH.

4. A compound as claimed in claim 1, wherein $R^1$ is $CH_3$, $R^2$ and $R^3$ are hydrogen and Y is —COOH.

5. A compound as claimed in any one of the preceding claims, which is in the E form where the group Y is trans to the indole ring.

6. A process for preparing a compound of the formula (I) as claimed in claim 1, which comprises reacting a compound of the formula:—

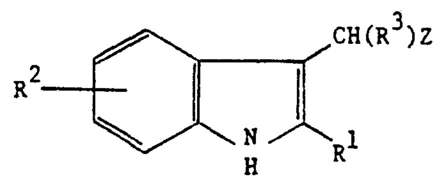

--- (II)

where $R^1$, $R^2$, $R^3$ and Z are as defined in claim 1,
with a compound of the formula:—

$$CH\equiv C.Y \qquad (III)$$

where Y is as defined in claim 1,
followed by, optionally, conversion of the product of the formula (I) into a pharmaceutically acceptable salt; or conversion of a compound of the formula (I) in which Y is —COOH into a compound in which Y is —$CONH_2$ by formation of an acid chloride or bromide or an imidazolide, followed by reaction with ammonia.

7. A process as claimed in claim 6, which is carried out in the presence of a base.

8. A process as claimed in claim 7, wherein said base is benzyltrimethylammonium hydroxide or tetrabutylammonium fluoride.

9. A pharmaceutical composition comprising a compound of the formula (I) or a pharmaceutically acceptable salt thereof as claimed in any one of claims 1 to 5, together with a pharmaceutically acceptable diluent or carrier.

10. A compound of the formula (I) or a pharmaceutically acceptable salt thereof as claimed in any one of claims 1 to 5 for use in treating an animal, including a human being, to inhibit the action of the thromboxane synthetase enzyme without significantly inhibiting the action of the prostacyclin synthetase or cyclo-oxygenase enzymes.

**Patentansprüche**

1. Verbindung der Formel:

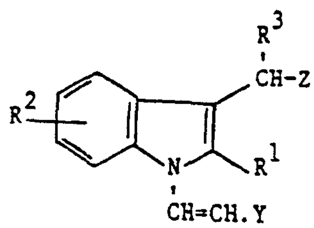

(I)

worin

$R^1$ H oder $C_1$—$C_4$-Alkyl ist,

$R^2$ H, $C_1$—$C_4$-Alkyl, $C_1$—$C_4$-Alkoxy oder Halogen ist,

$R^3$ H oder Methyl ist,

Y —COOH, —COO($C_1$—$C_4$-Alkyl) oder —$CONH_2$ ist und

Z 3-Pyridyl ist

und deren pharmazeutisch annehmbare Salze.

2. Verbindung nach Anspruch 1, worin $R^3$ Wasserstoff ist.

3. Verbindung nach Anspruch 1 oder 2, worin $R^1$ Methyl ist und Y —COOH ist.

4. Verbindung nach Anspruch 1, worin $R^1$ —$CH_3$ ist, $R^2$ und $R^3$ Wasserstoff sind und Y —COOH ist.

5. Verbindung nach einem der vorhergehenden Ansprüche, die in der E-Form ist, worin die Gruppe Y trans zu dem Indolring ist.

6. Verfahren zur Herstellung einer Verbindung der Formel (I) nach Anspruch 1, das das Umsetzen einer Verbindung der Formel:

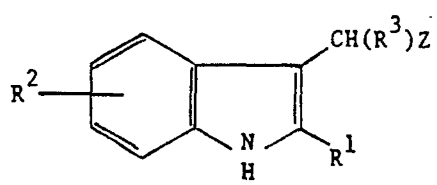

--- (II)

worin $R^1$, $R^2$, $R^3$ und Z der Definition in Anspruch 1 entsprechen, mit einer Verbindung der Formel:

$$CH \equiv C.Y \qquad (III)$$

worin Y der Definition in Anspruch 1 entspricht, umfaßt, gegebenenfalls gefolgt von der Umwandlung des Produktes der Formel (I) in ein pharmazeutisch annehmbares Salz, oder Umwandlung einer Verbindung der Formel (I), worin Y —COOH ist, in eine Verbindung, in der Y —$CONH_2$ ist, durch Bildung eines Säurechlorids oder -bromids oder eines Imidazolids, gefolgt von Umsetzung mit Ammoniak.

7. Verfahren nach Anspruch 6, das in Gegenwart einer Base durchgeführt wird.

8. Verfahren nach Anspruch 7, worin die Base Benzyltrimethylammoniumhydroxyd oder Tetrabutylammoniumfluorid ist.

9. Pharmazeutische Zusammensetzung enthaltend eine Verbindung der Formel (I) oder ein pharmazeutisch annehmbares Salz davon nach einem der Ansprüche 1—5 zusammen mit einem pharmazeutisch annehmbaren Verdünnungsmittel oder Träger.

10. Verbindung der Formel (I) oder ein pharmazeutisch annehmbares Salz davon nach einem der Ansprüche 1 bis 5 zur Verwendung bei der Behandlung eines tierischen Lebewesens, einschließlich des Menschen, um die Wirkung des Thromboxan-Synthetaseenzyms ohne bedeutende Inhibierung der Wirking der Prostacyclinsynthetase- oder Cyclooxygenaseenzyme zu hemmen.

**Revendications**

1. Composé de formule:

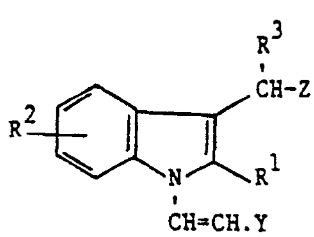

(I)

dans laquelle

$R^1$ représente H ou un groupe alkyle en $C_1$ à $C_4$;

$R^2$ représente H, un groupe alkyle en $C_1$ à $C_4$, alkoxy en $C_1$ à $C_4$ ou halogéno;

$R^3$ représente H ou un groupe méthyle;

Y représente un groupe —COOH, —COO(alkyle en $C_1$ à $C_4$) ou —$CONH_2$; et

Z est un groupe 3-pyridyle;

et leurs sels pharmaceutiquement acceptables.

2. Composé suivant la revendication 1, dans lequel $R^3$ est l'hydrogène.

3. Composé suivant la revendication 1 ou 2, dans lequel $R^1$ est un groupe méthyle et Y est un groupe —COOH.

4. Composé suivant la revendication 1, dans lequel $R^1$ est un groupe $CH_3$, $R^2$ et $R^3$ sont de l'hydrogène et Y est un groupe —COOH.

5. Composé suivant l'une quelconque des revendications précédentes, qui est sous la forme E dans laquelle le groupe Y a la configuration trans par rapport au noyau d'indole.

6. Procédé de préparation d'un composé de formule (I) suivant la revendication 1, qui consiste à faire réagir un composé de formula:

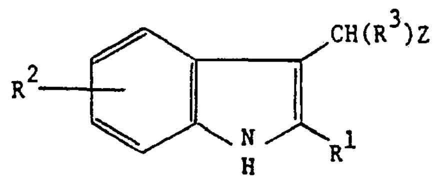

--- (II)

dans laquelle $R^1$, $R^2$, $R^3$ et Z sont tels que définis dans la revendication 1,
avec un composé de formule:

$$CH{\equiv}C.Y \qquad (III)$$

dans laquelle Y a la définition donnée dans la revendication 1,
la réaction étant suivie, facultativement, de la transformation du produit de formule (I) en un sel pharmaceutiquement acceptable; ou de la transformation d'un composé de formule (I) dans laquelle Y est un groupe —COOH en un composé dans lequel Y est un groupe —$CONH_2$ par formation d'un chlorure ou bromure d'acide ou d'un imidazolide, suivie de la réaction avec l'ammoniac.

7. Procédé suivant la revendication 6, qui est mis en oeuvre en présence d'une base.

8. Procédé suivant la revendication 7, dans lequel la base est l'hydroxyde de benzyltriméthyl-ammonium ou le fluorure de tétrabutylammonium.

9. Composition pharmaceutique comprenant un composé de formule (I) ou un sel pharmaceutiquement acceptable de ce composé suivant l'une quelconque des revendications 1 à 5 en association avec un diluant ou support pharmaceutiquement acceptable.

10. Composé de formule (I) ou sel pharmaceutiquement acceptable de ce composé suivant l'une quelconque des revendications 1 à 5, destiné à être utilisé dans le traitement d'un animal, y compris un être humain, pour inhiber l'action de l'enzyme appelé thromboxane-synthétase sans inhiber notablement l'action des enzymes appelés prostacycline-synthétase ou cyclo-oxygénase.